(19) Europäisches Patentamt
European Patent Office
Office européen des brevets

(11) **EP 2 144 555 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**11.05.2022 Patentblatt 2022/19**

(45) Hinweis auf die Patenterteilung:
**30.05.2012 Patentblatt 2012/22**

(21) Anmeldenummer: **08749811.9**

(22) Anmeldetag: **28.04.2008**

(51) Internationale Patentklassifikation (IPC):
**A61B 5/00** (2006.01)    **A61B 5/026** (2006.01)

(52) Gemeinsame Patentklassifikation (CPC):
**A61B 5/14542; A61B 5/026; A61B 5/14539; A61B 5/1477**

(86) Internationale Anmeldenummer:
**PCT/EP2008/055189**

(87) Internationale Veröffentlichungsnummer:
**WO 2008/132205 (06.11.2008 Gazette 2008/45)**

(54) **VORRICHTUNG UND VERFAHREN ZUR TRANSKUTANEN BESTIMMUNG VON BLUTGASEN**

DEVICE AND METHOD FOR TRANSCUTANEOUS DETERMINATION OF BLOOD GASES

DISPOSITIF ET PROCÉDÉ DE DÉTERMINATION PAR VOIE TRANSCUTANÉE DE GAZ SANGUINS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **27.04.2007 EP 07107130**

(43) Veröffentlichungstag der Anmeldung:
**20.01.2010 Patentblatt 2010/03**

(73) Patentinhaber: **SENTEC AG**
**4106 Therwil (CH)**

(72) Erfinder:
• **HAYOZ, Josef**
**CH-4133 Pratteln (CH)**
• **WAGNER, Rolf**
**CH-4204 Himmelried (CH)**

(74) Vertreter: **Hepp Wenger Ryffel AG**
**Friedtalweg 5**
**9500 Wil (CH)**

(56) Entgegenhaltungen:
**GB-A- 1 461 345**

• **ENKEMA L JR ET AL: "Laser Doppler velocimetry vs heater power as indicators of skin perfusion during transcutaneous O2 monitoring." CLINICAL CHEMISTRY MAR 1981, Bd. 27, Nr. 3, März 1981 (1981-03), Seiten 391-396, XP002496134 ISSN: 0009-9147**

EP 2 144 555 B2

**Beschreibung**

Die Erfindung betrifft eine Vorrichtung zur transkutaten Bestimmung von Blutgasen gemäss dem Oberbegriff von Anspruch 1. Die Erfindung betrifft weiter ein Verfahren zur transkutanen Bestimmung von Blutgasen gemäss dem Oberbegriff von Anspruch 8.

**Stand der Technik**

Die Kenntnis der Konzentration von Blutgasen im arteriellen Blut, beziehungsweise des arteriellen Kohlendioxidpartialdrucks (PaCO2) und des arteriellen Sauerstoffpartialdrucks (PaO2), sind zur Bestimmung und Überwachung des respiratorischen Status eines Patienten von Bedeutung. Da sich der respiratorische Status eines Patienten sehr schnell ändern kann und insbesondere Hypoxien oder Hyperkapnien den Zustand des Patienten nachteilig beeinträchtigen können, ist eine kontinuierliche und idealerweise nicht-invasive Überwachung der Blutgase in vielen Fällen erforderlich.

Die Gase Kohlendioxid (CO2) und Sauerstoff (O2) haben die Eigenschaft, dass sie durch das Gewebe des Körpers und insbesondere durch die Haut diffundieren. Mit einem sogenannt transkutanen Sensor - einem geeigneten, auf der Hautoberfläche aufliegenden Sensor - ist es deshalb möglich, den Haut-Kohlendioxidpartialdruck (PsCO2) beziehungsweise den Haut-Sauerstoffpartialdruck (PsO2) im Bereich des Sensors nicht-invasiv und kontinuierlich zu messen, und daraus mittels geeigneter Verfahren einen sogenannten transkutanen Kohlendioxidpartialdruck (tcpCO2) beziehungsweise einen transkutanen Sauerstoffpartialdruck (tcpO2) zu bestimmen. Der für PsCO2 und PsO2 verwendete Index "s" hat die Bedeutung von Haut (skin).

Der transkutane Kohlendioxidpartialdruck (tcpCO2) beziehungsweise der transkutane Sauerstoffpartialdruck (tcpO2) sollte idealerweise derart bestimmt werden, dass dieser dem arteriellen Kohlendioxidpartialdruck (PaCO2) beziehungsweise dem arteriellen Sauerstoffpartialdruck (PaO2) entspricht. Bislang treten zwischen diesen Werten oft erhebliche Differenzen auf, was leider bedeutet, dass die transkutane Bestimmung von Blutgasen oft fehlerhaft ist.

Die Druckschrift WO 02/41770 offenbart derartige Vorrichtungen und Verfahren, beispielsweise zur Bestimmung des transkutanen Kohlendioxidpartialdrucks (tcpCO2) nach Stow-Severinghaus oder des transkutanen Sauerstoffpartialdruck (tcpO2) nach Clark. Der dabei verwendete, an der Haut aufliegende transkutane Sensor verfügt neben den zur Messung des Haut-Kohlendioxidpartialdrucks (PsCO2) beziehungsweise des Haut-Sauerstoffpartialdrucks (PsO2) verwendeten CO2- beziehungsweise O2-Sensoren auch über ein Heizelement, welches die Haut im Bereich des Sensors typischerweise auf eine konstante Temperatur (Ts) erwärmt, welche höher liegt als die übliche Körperoberflächentemperatur.

Die nachfolgende, von Severinghaus vorgeschlagene Gleichung (1), wird verwendet, um aus dem bei der Haut-Temperatur Ts gemessenen Haut-Kohlendioxidpartialdruck (PsCO2(Ts)) den transkutanen Kohlendioxidpartialdruck für eine gegebene Referenztemperatur Tr zu bestimmen:

$$tcpCO2(Tr) = \frac{PsCO2(Ts)}{10^{(Ts-Tr) \times A}} - Ms \qquad (1)$$

Wobei die verwendeten Parameter folgende Bedeutung haben:

| | |
|---|---|
| Ts: | Temperatur der Haut im Bereich des Sensors |
| Tr: | Referenztemperatur, typische 37°C |
| PsCO2(Ts): | Der bei der Temperatur Ts im Bereich des Sensors vorliegende Haut-Kohlendioxidpartialdruck. |
| Ms: | Der Metabolische Offset. |
| A: | Anärober Temperaturfaktor |

Der erste Term der Gleichung (1) korrigiert den bei einer Haut-Temperatur von Ts gemessenen Wert von PsCO2(Ts) auf die Referenz-Temperatur Tr, unter Verwendung des anäroben Temperaturfaktors (A). Die als metabolischer Offset bezeichnete Konstante Ms trägt der verbleibenden Differenz zwischen dem Haut-Kohlendioxidpartialdruck und dem arteriellen Kohlendioxidpartialdruck Rechnung.

Die vorhin genannte Gleichung (1) ist aus der Literatur auch in der nachfolgenden, leicht modifizierten Form bekannt:

$$tcpCO2(Tr) = \frac{PsCO2(Ts) - Ms}{10^{(Ts-Tr) \times A}}$$

Die nachfolgende, von Clark vorgeschlagene Gleichung (2), wird verwendet, um aus dem bei der Haut-Temperatur Ts gemessenen Haut-Sauerstoffpartialdruck (PsO2(Ts)) den transkutanen Sauerstoffpartialdrucks für eine gegebene Referenztempertur Tr zu bestimmen:

$$tcpO_2(Tr) = Corr * PsO_2(Ts) \qquad (2)$$

Wobei der verwendete Parameter folgende Bedeutung hat:
Corr: Korrekturfaktor

Nachteilig an bekannten Sensoren beziehungsweise an dem in Gleichung (1) dargestellten Korrekturverfahren ist die Tatsache, dass zwischen dem mittels Gleichung (1) bestimmten transkutanen Kohlendioxidpartialdruck (tcpCO2(Tr)) und dem im arteriellen Blut bei der Referenztemperatur Tr effektiv vorhandenen Kohlendioxidpartialdruck (PaCO2(Tr)) erhebliche Abweichungen auftreten können. Dasselbe gilt für den mittels Gleichung (2) bestimmten transkutanen Sauerstoffpartialdruck (tcpO2(Tr)), welcher ebenfalls erhebliche Abweichungen zum bei der Referenztemperatur Tr effektiv im arteriellen Blut vorhandenen Sauerstoffpartialdruck (PaO2(Tr)) aufweisen kann.

Das Dokument GB 1 461 345 offenbart eine Verfahren sowie eine Vorrichtung zum Bestimmen eines Perfusionseffizienzfaktors (perfusion efficiency factor) von tierischem Gewebe. Nach der Bestimmung des Perfusionseffizienzfaktors wird die Blutzirkulation lokal unterbrochen, und danach der Druckabfall des Blutsauerstoffdruckes P02 in Funktion der Zeit gemessen. Dieses Verfahren ermöglicht den Perfusionseffizienzfaktor mit weniger Fehlern zu bestimmen. Dieses bekannte Verfahren sowie diese bekannte Vorrichtung weisen die Nachteile auf, dass diese nicht geeignet sind einen transkutanen Kohlendioxidpartialdruck (tcpCO2) und einen transkutanen Sauerstoffpartialdruck (tcpO2) genau zu bestimmen. Zudem ist das Verfahren nur für Tiere geeignet. Das Verfahren erfordert zudem, dass die Blutzirkulation lokal unterbrochen wird, was für einen Menschen höchst unangenehm wäre.

**Darstellung der Erfindung**

Es ist daher Aufgabe der vorliegenden Erfindung, eine Vorrichtung sowie ein Verfahren vorzuschlagen, um die Übereinstimmung zwischen dem transkutanen Kohlendioxidpartialdruck (tcpCO2(Tr)) und dem arteriellen Kohlendioxidpartialdruck (PaCO2(Tr)) und bevorzugt zwischen dem transkutanen Sauerstoffpartialdruck (tcpO2(Tr)) und dem arteriellen Sauerstoffpartialdruck (PaO2(Tr)) zu verbessern.

Diese Aufgabe wird gelöst mit einer Vorrichtung aufweisend die Merkmale von Anspruch 1. Die Unteransprüche 2 bis 7 beziehen sich auf weitere, bevorzugte Ausführungsformen der Vorrichtung. Die Aufgabe wird weiter gelöst mit einem Verfahren aufweisend die Merkmale von Anspruch 8. Die Unteransprüche 9 bis 11 beziehen sich auf weitere, bevorzuge Verfahrensschritte.

Vergleichsstudien haben insbesondere gezeigt, dass Schwankungen in der lokalen Gewebedurchblutung und insbesondere eine geringe lokale Gewebedurchblutung eine erhöhte Abweichung zwischen dem ermittelten transkutanen Kohlendioxidpartialdruck (tcpCO2) und der effektiven arterielle Kohlendioxidkonzentration (PaCO2) zur Folge haben. Dasselbe gilt für den ermittelten transkutanen Sauerstoffpartialdruck (tcpO2) und die effektive arterielle Sauerstoffkonzentration (Pa02). Um aus dem gemessenen Haut-Kohlendioxidpartialdruck (PsCO2) zuverlässig die effektive arterielle Kohlendioxidkonzentration (PaCO2) zu ermitteln, ist es daher erfindungsgemäss erforderlich, bei der Berechnung des transkutanen Kohlendioxidpartialdrucks (tcpCO2) einen Durchblutungskorrekturfaktor F zu verwenden. Der Wert F ist dabei vorzugsweise nahe am transkutanen Sensor zu messen, vorzugsweise unterhalb des transkutanen Sensors, zumindest jedoch vorzugsweise in einem Umfeld von nicht mehr als 1 bis 2 cm von der Auflagefläche des transkutanen Sensors entfernt. Um aus dem gemessenen Haut- Sauerstoffpartialdruck (Ps02) zuverlässig die effektive arterielle Sauerstoffkonzentration (Pa02) zu ermitteln, kann es daher vorteilhaft sein, bei der Berechnung des transkutanen Sauerstoffpartialdruck (tcpO2) einen Durchblutungskorrekturfaktor F zu verwenden.

Ausgehend vom dem bei der Haut-Temperatur Ts gemessenen Haut-Kohlendioxidpartialdruck (PsCO2(Ts)) wird der transkutane Kohlendioxidpartialdruck für eine gegebene Referenztemperatur Tr wie folgt in Funktion des Wertes F berechnet (Gleichung 1"):

$$tcpCO2(Tr,F) = \frac{PsCO2(Ts)}{10^{(Ts-Tr) \times A}} - Ms(Ts,F) \qquad (1'')$$

| Ts: | Temperatur der Haut im Bereich des Sensors |
|---|---|
| Tr: | Referenztemperatur, typische 37°C |
| PsCO2(Ts): | Der bei der Temperatur Ts im Bereich des Sensors vorliegende Haut-Kohlendioxidpartialdruck |
| Ms(Ts,F): | Der bei der Temperatur Ts im Bereich des Sensors vorliegende Metabolische Offset in Funktion des Wertes F. Der Metabolische Offset wird in Abhängigkeit von Ts korrigiert. |
| A: | Anärober Temperaturfaktor |

Ausgehend vom dem bei der Haut-Temperatur Ts gemessenen Haut-Sauerstoffpartialdruck (PsO2(Ts)) kann der transkutane Sauerstoffpartialdruck für eine gegebenen Referenztemperatur Tr wie folgt in Funktion des Wertes F berechnet werden (Gleichung 2"):

$$tcpO2(Tr, F) = Corr(Tr, Ts, F) * PsO2(Ts) \qquad (2'')$$

Wobei der verwendete Parameter folgende Bedeutung hat:

Corr (Tr,Ts, F): Von der Referenztemperatur Tr, von der im Bereich des Sensors vorherrschenden Hauttemperatur Ts und vom Wert F abhängiger Korrekturfaktor. In einer vereinfachten Version wird der Korrekturfaktor nur in Funktion des Wertes F korrigiert, sodass in einer vereinfachten Gleichung 2" nur der Faktor Corr (F) berücksichtigt wird.

Ein wesentlicher Vorteil der erfindungsgemässen Vorrichtung beziehungsweise des erfindungsgemässen Verfahrens ist darin zu sehen, dass der Einfluss der Durchblutung reduziert oder eliminiert wird, welcher zu einer Abweichung zwischen dem transkutanen Kohlendioxidpartialdruck (tcpCO2(Tr)) und dem arteriellen Kohlendioxidpartialdruck (PaCO2(Tr)) und bevorzugt auch zwischen dem transkutanen Sauerstoffpartialdruck (tcpO2(Tr)) und dem arteriellen Sauerstoffpartialdruck (PaO2(Tr)) geführt hat. Der Wert F bezeichnet vorzugsweise den Blutfluss, wobei der Wert F auch eine andere Messgrösse sein kann, welche als ein Mass für die lokale Gewebedurchblutung beziehungsweise für die lokale Perfusion der Haut dienen kann. Es sind insbesondere Messgrössen von Interesse, welche die lokale Verfügbarkeit von Gasen berücksichtigen, insbesondere die Verfügbarkeit von arteriellen Gasen, die zugeströmte Blutmenge, oder die abtransportierte Blutmenge, oder die Pulsation. Unter dem Begriff "Gewebedurchblutung" wird somit ein messbarer Wert verstanden, der insbesondere die lokale Verfügbarkeit beziehungsweise die lokal vorhandene Menge von arteriellen Gasen zu erfassen erlaubt. Dank der Kenntnis der Gewebedurchblutung ist es möglich die gemessenen Werte derart zu korrigieren, dass der ermittelte Wert für den transkutanen Kohlendioxidpartialdruck (tcpCO2(Tr)) und bevorzugt den transkutanen Sauerstoffpartialdruck (tcpO2(Tr)) nicht mehr oder nur noch geringfügig von der Gewebedurchblutung abhängt. Mit anderen Worten ermöglicht die erfindungsgemässe Vorrichtung beziehungsweise das erfindungsgemässe Verfahren eine perfusionsabhängige beziehungsweise durchblutungsabhängige Korrektur, um dadurch den durch die Perfusion beziehungsweise die Gewebedurchblutung bewirkten Einfluss auf die ermittelten Werte zu reduzieren oder zu eliminieren. Es ist eine Mehrzahl von Möglichkeiten bekannt, die Perfusion oder Durchblutung, einen der Perfusion oder Durchblutung äquivalenten oder ähnlichen Wert, beziehungsweise den Wert F der Gewebedurchblutung zu messen. So könnte beispielsweise die Puls-Volumen Modulation gemessen werden, auch als "Totale Pulsmodulation TPM" bezeichnet, um daraus einen Wert F zu berechnen. Der Fluss F, das heisst beispielsweise das Volumen pro Sekunde, kann auf unterschiedlichste Weise gemessen werden. Wichtig ist, dass der Fluss F lokal, das heisst möglichst in der Nähe oder unterhalb des transkutanen Sensors gemessen wird. Mit den nachfolgend beispielhaft genannten Methoden kann der Fluss F beispielsweise unterhalb des transkutanen Sensors gemessen werden:

1. Dopplermessung: Der Fluss beziehungsweise die Gewebedurchblutung F des Blutes kann unter Nutzung des Dopplereffektes bestimmt werden, zum Beispiel durch eine optische oder akustische Messung.
2. (Photo)Plethysmographisches Messsystem: Der Fluss beziehungsweise die Gewebedurchblutung F des Blutes kann mit einem (Photo)Plethysmographischen Messsystem gemessen werden, insbesondere mit einem pulsspektroskopischen Messsystem. Zur Bestimmung eines Masses für die Gewebedurchblutung F kann bei einer photoplethysmographischen Messung der Wechselspannungs- und/oder der Gleichspannungsanteil der gemessenen Lichtsignale verwendet werden.
3. Spektroskopische Messung: Die Gewebedurchblutung F kann auch optisch durch Messung des Spektrums, insbesondere in nahen Infrarot (NIR) gemessen werden, beispielsweise mit einer Vorrichtung und einem Verfahren wie dies in der Druckschrift US 2005/0277818 A1 offenbart ist.

4. Heizenergie: Messung der Heizenergie, welche erforderlich ist, um eine an der Haut aufliegende Vorrichtung auf einer konstanten Temperatur zu halten, wobei diese Temperatur höher ist als die Hauttemperatur. Es gilt, dass bei grösserem Fluss F eine höhere Heizenergie erforderlich ist, um die Temperatur konstant zu halten, sodass über die erforderliche Heizenergie der Fluss F abgeschätzt werden kann.

Abhängig von der gewählten Messmethode kann der Wert F auch für schwierige, klinisch jedoch bedeutsame Situationen wie arterielle Hypotonie, Hypovolämie nach Blutungsverlusten des Patienten oder Vasokonstriktion der peripheren kleinen Arterien gemessen werden. Diese drei beschriebenen Zustände sind klinisch häufig, insbesondere in der intra- und postoperativen Applikation. Bei diesen Zuständen ist am Applikationsort Blut zu finden, wobei dieses jedoch nicht mehr hinreichend durch einen Blutimport aufgefrischt wird. Auch eine derartige Situation kann mit dem Wert F erfasst werden. Ein derartiger Zustand kann beispielsweise mit einem photoplethysmographischen Messsystem erfasst werden, das aus einem detektierten Lichtsignal ein Wechselspannungssignal und ein Gleichspannungssignal ableitet. Es ist zu berücksichtigen, dass die erfindungsgemässe Vorrichtung beziehungsweise das erfindungsgemässe Verfahren die lokale Perfusion nicht absolut sondern nur näherungsweise ermitteln kann. Diese ermittelte, lokale Perfusion erlaubt es jedoch die Messqualität von transkutan ermittelten Blutgaswerten erheblich zu verbessern. Der Wert F könnte noch einen weiteren Korrekturfaktor umfassen, nämlich lokale Hauteigenschaften, weil die Korrektur nebst der Durchblutung auch noch von weiteren lokalen Hauteigenschaften abhängen kann. Diese lokalen Hauteigenschaften können beispielsweise über das Gleichspannungssignal des photoplethysmographischen Messsystems gemessen werden, da mit diesem Messsignal die gesamte Absorptionsfähigkeit des Gewebes gemessen werden kann, nicht nur der Anteil des anströmenden Hämoglobins. Dieses Messsignal ist daher vorzugsweise ein Mass für die gesamte optische Dichte und erlaubt daher einen Rückschluss auf die Histoanatomie des Messortes. Ein derart bestimmter beziehungsweise korrigierter Wert F ermöglicht es, die Messqualität von transkutan ermittelten Blutgaswerten erheblich zu verbessern.

## Kurze Beschreibung der Zeichnungen

Die zur Erläuterung der Ausführungsbeispiele verwendeten Zeichnungen zeigen:

Fig. 1  einen Längsschnitt durch einen bekannten transkutanen Sensor;
Fig. 2  eine Draufsicht auf den in Figur 1 dargestellten transkutanen Sensor;
Fig. 3  einen Längsschnitt durch einen auf der Haut aufliegenden transkutanen Sensor;
Fig. 4  eine grafische Darstellung der Korrektur des metabolischen Offsets Ms in Funktion der Gewebedurchblutung F;
Fig. 5  einen grafische Darstellung des zeitlichen Verlaufs des mittels Blutgasanalyse bestimmten arteriellen Kohlendioxidpartialdrucks (Kurve a) sowie des mittels Gleichung 1 (unkorrigiert, Kurve b) beziehungsweise mittels Gleichung 1" (fluss-korrigierten, Kurve c) transkutanen Kohlendioxidpartialdrucks. Alle Kurven sind für die gleiche Referenztemperatur (Tr = 37°C) dargestellt.

Grundsätzlich sind in den Zeichnungen gleiche Teile mit gleichen Bezugszeichen versehen.

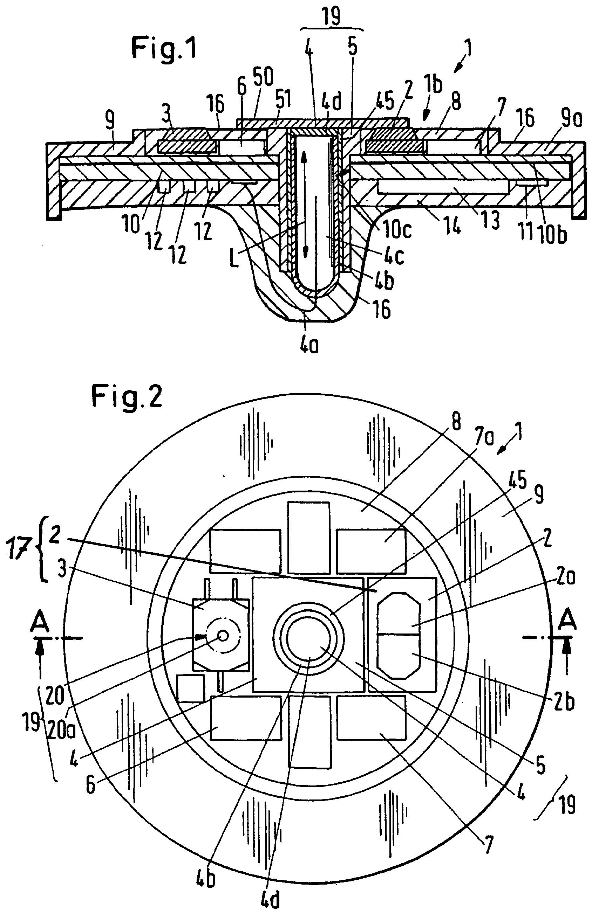

Der in den Figuren 1 und 2 dargestellte Sensor 1 ist aus der Druckschrift WO 02/41770 bekannt. Der dargestellte Sensor 1 erlaubt eine kombinierte Messung der arteriellen Sauerstoffsättigung (SpO2) und des transkutanen CO2-Partialdrucks (tcpCO2). Zur Messung der Sauerstoffsättigung weist der Sensor 1 ein pulsoximetrisches Messsystem 17 auf, welches unter anderem eine zweifarbige Leuchtdiode 2 (LED) sowie einen Fotodetektor 3 umfasst. Die zweifarbige Leuchtdiode 2 umfasst zwei dicht nebeneinander liegende, in einem gemeinsamen Gehäuse angeordnete Leuchtdioden 2a, 2b, wobei die eine Leuchtdiode 2a eine Wellenlänge von etwa 660 nm (Rot) und die andere Leuchtdiode 2b eine Wellenlänge von etwa 890 nm (Infrarot) aufweist. Der Sensor 1 weist eine Oberfläche 1b auf, über welcher im dargestellten Ausführungsbeispiel eine Membran 50 und dazwischen ein dünne Schicht Elektrolyt 51 angeordnet ist. Diese Membran 50 wird an einer gut durchbluteten Stelle des menschlichen Körpers auf die Haut aufgelegt, zum Beispiel an einem Finger, an der Stirn oder am Ohrläppchen. Das von den beiden Leuchtdioden 2a,2b ausgestrahlte Licht durchstrahlt den sich über den Leuchtdioden 2a,2b befindlichen Elektrolyt 51 sowie die Membran 50, und wird in das nicht dargestellte, gut durchblutete Körperteil geleitet und dort gestreut und teilweise absorbiert. Das vom Körperteil reflektierte Licht wird mit dem Fotodetektor 3 gemessen. Das vom Fotodetektor 3 gemessene Signal wird einem digitalen Sensorsignalprozessor 13 zugeleitet.

Der dargestellte Sensor 1 umfasst zudem eine elektrochemische Messvorrichtung 19 zur Messung des transkutanen Kohlendioxidpartialdrucks tcpCO2-Messung, wobei diese Messvorrichtung 19 eine Mikro-pH-Elektrode 4 sowie eine Ag/AgCl-Referenzelektrode 5 umfasst. Der transkutane Kohlendioxidpartialdruck wird potentiometrisch gemessen, indem der pH-Wert der dünnen Schicht der Elektrolytlösung 51 gemessen wird, welche über die gut gasdurchlässige, hydrophobe Membran 50 mit der Haut in Verbindung steht. Eine Änderung des pCO2-Wertes an der Hautoberfläche bewirkt eine pH-Änderung der Elektrolytlösung, die sich proportional zum Logarithmus der pCO2-Änderung verhält. Der pH-Wert wird gemessen indem das Potential zwischen der Miniatur-pH-Elektrode 4 und der Ag/AgCl-Referenzelektrode 5 gemessen wird. Die Mikro-pH-Elektrode 4 ist über den elektrischen Innenableiter 4a Signal leitend mit dem digitalen Sensorsignalprozessor 13 verbunden.

Der dargestellte Sensor 1 umfasst zudem ein Erwärmungssystem 18 umfassend eine als elektrischer Widerstand ausgestaltete Erwärmungsvorrichtung 6 sowie einen Temperatursensor 7 zur Temperaturregelung. Das Erwärmungssystem 18 wird vorteilhafterweise in Kombination mit der elektrochemischen Messvorrichtung 19 verwendet, um über die Sensoroberfläche 1b die sich darunter befindliche Haut zu erwärmen. Zur transkutanen Messung des Kohlendioxidpartialdrucks pCO2 oder des Sauerstoffpartialdrucks pO2 wird die Sensoroberfläche 1b beispielsweise auf eine Temperatur von etwa 40 °C bis 44 oder 45°C erwärmt.

Der Sensor 1 umfasst eine mehrschichtige, starre Leiterplatte 10, welche mit elektronischen Komponenten 2,3,6,7,12,13 bestückt ist, und welche eine Vielzahl nicht dargestellte elektrische Leiterbahnen aufweist, um die elektronischen Komponenten wie die Leuchtdiode 2, den Fotodetektor 3, den Widerstand 6, den Temperatursensor 7, einen zweiten Temperatursensor 7a oder weitere elektronische Komponenten wie Verstärker 12, 12a Signal leitend zu verbinden.

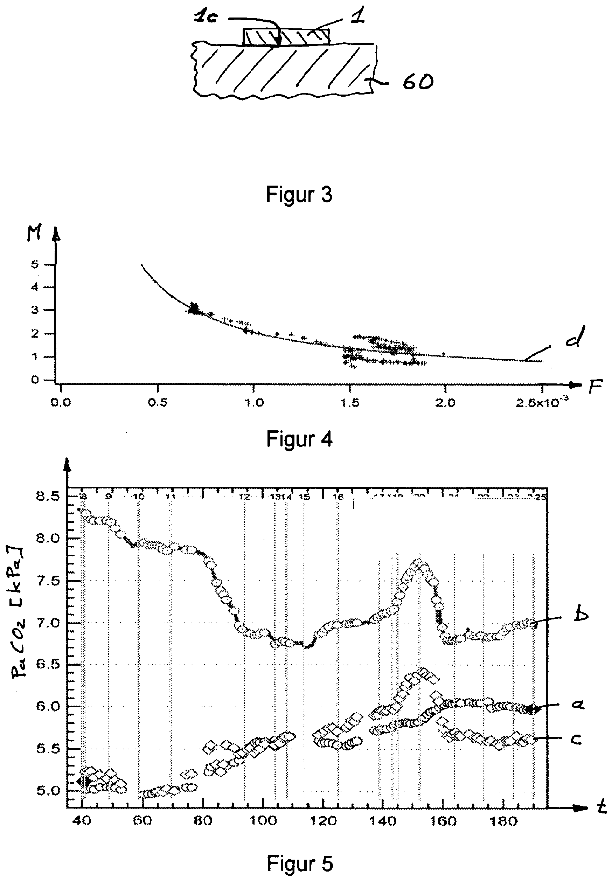

Figur 3 zeigt einen Längsschnitt durch einen auf der Haut 60 aufliegenden Sensor 1. Der Sensor zum Messen der lokalen Gewebedurchblutung F ist vorzugsweise derart ausgestaltet ist, dass dieser unterhalb der Auflagefläche des transkutanen Sensors beziehungsweise unterhalb der Auflagefläche 1c des ganzen Sensors 1 die lokale Gewebedurchblutung F misst. Bevorzugt wird die lokale Gewebedurchblutung F etwa in einem Bereich von bis zu 4 cm Entfernung vom Sensor 1, und vorzugsweise in einem Bereich von bis zu 2 cm Entfernung von der Auflagefläche 1c des Sensors 1 gemessen.

Der in Figur 1 dargestellte Sensor 1 weist ein pulsoximetrisches Messsystem 17 auf, welches bisher zur Messung der Sauerstoffsättigung verwendet wurde. Das pulsoximetrische Messsystem 17 kann jedoch auch, zur Messung der Gewebedurchblutung F verwendet werden. Das in Figur 1 dargestellte pulsoximetrische Messsystem 17 ist in der Lage über das von der zweifarbigen Leuchtdiode 2 abgestrahlte und in der Haut reflektierte Licht, welches vom Fotodetektor 3 gemessen wird, durch eine entsprechende Berechnung die Gewebedurchblutung F zu ermitteln. Dadurch kann die lokale Gewebedurchblutung F unterhalb der Auflagefläche 1a des Sensors 1 bestimmt werden.

Die lokale Gewebedurchblutung F kann beispielsweise auch mit einer Erwärmungsvorrichtung bestimmt werden, indem diese beispielsweise die Temperatur der Sensor-Auflagefläche konstant hält, wobei die der Erwärmungsvorrichtung zugeführte Leistung ein Mass für die Gewebedurchblutung F darstellt.

Figur 4 zeigt den Zusammenhang des metabolischen Offsets Ms in Funktion der lokalen Gewebedurchblutung F, wobei F im dargestellten Ausführungsbeispiel pulsoximetrisch, zum Beispiel mit einem wie in Figur 1 und 2 dargestellten Sensor, bestimmt wurde. Die lokale Gewebedurchblutung könnte mit einer anderen Vorrichtung auch pulsspektroskopisch gemessen werden. Wird zudem noch, wie gemäss der Erfindung in Gleichung 1" angedeutet, die Temperatur Ts berücksichtigt, so ergäbe sich in Figur 4 eine Kurvenschar von im Wesentlichen insbesondere in Funktion der Temperatur Ts in vertikaler Richtung gegeneinander verschobenen Kurven.

Figur 5 zeigt verschiedene Kohlendioxidpartialdruckkurven. Die Kurve a zeigt den effektiv im Blut vorhandenen, mittels arterieller Blutgasanalyse für eine Referenztemperatur von 37°C bestimmten Kohlendioxidpartialdruck (PaCO2(37°C)). Die Kurve b beziehungsweise c stellen den zeitlichen Verlauf des - ausgehend von dem bei 42°C mit dem in Figur 1 und 2 dargestellten Sensor 1 gemessenen Haut-Kohlendioxidpartialdruck (PsCO2(42°C)) - mit Gleichung 1 beziehungsweise mit Gleichung 1" (ohne Berücksichtigung einer Temperaturkorrektur beim metabolischen Offset Ms) für eine Referenztemperatur von 37°C berechneten transkutanen Kohlendioxidpartialdrucks (tcpCO2(37°C)) dar. Bei der Berechnung mittels Gleichung 1" (Kurve c) wurde die in Figur 4 dargestellt Abhängigkeit des Metabolischen Offset Ms von der lokalen Gewebedurchblutung F verwendet. Wie aus Figur 5 ersichtlich, weicht der Verlauf von Kurve b erheblich vom Verlauf der Kurve a ab, während der Verlauf der Kurve c im Wesentlichen dem Verlauf der Kurve a entspricht. Dies bedeutet, dass der zeitliche Verlauf des mittels Gleichung 1" bestimmten transkutanen Kohlendioxidpartialdrucks (tcpCO2(37°C)) sehr gut mit dem zeitlichen Verlauf des effektiven Kohlendioxidpartialdrucks (PaCO2(37°C)) übereinstimmt. Die erfindungsgemässe Vorrichtung, beziehungsweise das erfindungsgemässe Verfahren, erlaubt es somit den Verlauf des Kohlendioxidpartialdrucks PaCO2 sehr genau zu bestimmen. Die Berücksichtigung der Gewebedurchblutung F zur Korrektur gemessener Werte ist insbesondere dann von Wichtigkeit, wenn die Durchblutung der Haut unterhalb des Sensors gering ist, da dann das durch den Metabolismus produzierte CO2 nicht mehr effizient durch das Blut abtransportiert werden kann. Das erfindungsgemässe Verfahren weist somit den Vorteil auf, dass die Konzentration von Blutgasen im arteriellen Blut auch bei Patienten mit Durchblutungsstörungen, geringer Durchblutung oder veränderlicher Durchblutung sicher und zuverlässig gemessen werden kann. Die erfindungsgemässe Vorrichtung beziehungsweise das erfindungsgemässe Verfahren ermöglicht es somit, auch bezüglich Kreislauf und Durchblutung schwierige und daher bezüglich Überwachung sehr anspruchsvolle Patienten sicher und zuverlässig zu überwachen.

**Patentansprüche**

1. Vorrichtung zur transkutanen Bestimmung von Blutgasen umfassend einen transkutanen Sensor zur Messung der Grösse Haut-Kohlendioxidpartialdruck (PsCO2), umfassend mindestens einen Sensor zum Messen der bezüglich des transkutanen Sensors lokalen Gewebedurchblutung (F), sowie umfassend eine Vorrichtung zum Berechnen der Grösse transkutaner Kohlendioxidpartialdruck (tcpCO2) aus dem gemessenen Haut-Kohlendioxidpartialdruck (PsCO2), wobei bei der Berechnung von der Grösse transkutaner Kohlendioxidpartialdruck (tcpCO2) ein von der lokalen Gewebedurchblutung (F) abhängiger Faktor berücksichtigt wird, **dadurch gekennzeichnet, dass** die Bestimmung des transkutanen Kohlendioxidpartialdruckes (tcpCO2) unter Berücksichtigung der lokalen Gewebedurchblutung (F) und zusätzlich der lokalen Temperatur (Ts) entsprechend der Gleichung

$$tcpCO2(Tr, F) = \frac{PsCO2(Ts)}{10^{(Ts-Tr) \times A}} - Ms(Ts, F)$$

erfolgt.

2. Vorrichtung nach Anspruch 1, wobei der transkutane Sensor zur Messung der Grösse Haut-Kohlendioxidpartialdruck (PsCO2) zur Messung der Grösse Haut Sauerstoffpartialdruck (PsO2) ausgebildet ist und wobei die Vorrichtung zum Berechnen der Grösse transkutaner Kohlendioxidpartialdruck (tcpCO2) aus dem gemessenen Haut-Kohlendioxid-partialdruck (PsCO2) zum Berechnen der Grösse transkutaner Sauerstoffpartialdruck (tcpO2) aus dem gemessenen Haut-Sauerstoffpartialdruck (PsO2) ausgebildet ist, wobei bei der Berechnung der Grösse transkutaner Sauerstoffpartialdruck (tcpO2) ein von der lokalen Gewebedurchblutung (f) abhängiger Faktor berücksichtigt wird, **dadurch gekennzeichnet, dass** die Bestimmung des transkutanen Sauerstoffpartialdruckes (tcpO2) unter Berücksichtigung der lokalen Gewebedurchblutung (F) entsprechend der Gleichung tcpO2 (Tr, F) = Corr(F) * PsO2 (Ts) erfolgt.

3. Vorrichtung nach Anspruch 2, **dadurch gekennzeichnet, dass** die Bestimmung des transkutanen Sauerstoffpartialdruckes (tcp02) unter Berücksichtigung der lokalen Temperatur (Ts) entsprechend der Gleichung tcpO2 (Tr, F) = Corr(Tr, Ts, F) * PsO2 (Ts) erfolgt.

4. Vorrichtung nach einem der vorhergehenden Ansprüche, **dadurch gekennzeichnet, dass** zumindest ein Sensor zum Messen der lokalen Gewebedurchblutung (F) in demselben Gehäuse angeordnet ist wie der transkutane Sensor, und/oder dass Sensoren zum Messen der lokalen Gewebedurchblutung (F) derart ausgestaltet sind, dass diese unterhalb der Auflagefläche des transkutanen Sensors die lokale Gewebedurchblutung (F) messen.

5. Vorrichtung nach einem der vorhergehenden Ansprüche, **dadurch gekennzeichnet, dass** Sensoren zum Messen der lokalen Gewebedurchblutung (F) Teil eines (photo)pletysmographischen, insbesondere eines pulsspektroskopischen oder eines pulsoximetrischen Messsystems sind.

6. Vorrichtung nach einem der Ansprüche 1 bis 4, **dadurch gekennzeichnet, dass** Sensoren zum Messen der lokalen Gewebedurchblutung (F) Teil einer Erwärmungsvorrichtung sind, welche die Temperatur der Auflagefläche konstant halten, wobei die der Erwärmungsvorrichtung zugeführte Leistung ein Mass für die Gewebedurchblutung (F) darstellt.

7. Vorrichtung nach einem der Ansprüche 1 bis 4, **dadurch gekennzeichnet, dass** Sensoren zum Messen der lokalen Gewebedurchblutung (F) als akustische Sensoren oder Lichtsensoren, insbesondere Lasersensoren, ausgestaltet sind, und Teil eines Dopplermesssystems bilden.

8. Verfahren zur transkutanen Blutgasüberwachung wobei die Grösse Haut-Kohlendioxidpartialdruck (PsCO2) erfasst wird, und wobei eine lokale Gewebedurchblutung (F) erfasst wird, und wobei mit einer Berechnungsvorrichtung die Grössen transkutaner Kohlendioxidpartialdruck (tcpCO2) aus dem gemessenen Haut-Kohlendioxidpartialdruck (PsCO2) berechnet wird, wobei bei der Berechnung der Grösse transkutaner Kohlendioxidpartialdruck (tcpCO2) die lokale Gewebedurchblutung (F) und zusätzlich die lokale Temperatur (Ts) berücksichtigt wird, **dadurch gekennzeichnet, dass** der transkutane Kohlendioxidpartialdruck (tcpCO2) in Funktion der Gewebedurchblutung (F) entsprechend der Gleichung

$$tcpCO2(Tr, F) = \frac{PsCO2(Ts)}{10^{(Ts-Tr) \times A}} - Ms(Ts, F)$$

berechnet wird.

9. Verfahren nach Anspruch 8, wobei die Grösse Haut-Sauerstoffpartialdruck (PsO2) erfasst wird und wobei mit der Berechnungsvorrichtung für die Berechnung der Grösse transkutaner Kohlendioxidpartialdruck (tcpCO2) aus dem gemessenen Haut- Kohlendioxidpartialdruck (PsC02) die Grösse transkutaner Sauerstoffpartialdruck (tcpO2) aus dem gemessenen Haut-Sauerstoffpartialdruck (PsO2) berechnet wird, wobei bei der Berechnung von der Grösse transkutaner Sauerstoffpartialdruck (tcpO2) die lokale Gewebedurchblutung (F) berücksichtig wird, **dadurch gekennzeichnet, dass** der transkutane Sauerstoffpartialdruck (tcpO2) in Funktion der Gewebedurchblutung (F) entsprechend der Gleichung tcpO2 (Tr, F) = Corr(Tr, Ts, F) * PsO2 (Ts) berechnet wird.

10. Verfahren nach Anspruch 9, **dadurch gekennzeichnet, dass** der transkutane Sauerstoffpartialdruck (tcpO2) zusätzlich in Funktion der lokalen Temperatur (Ts) entsprechend der Gleichung tcpO2 (Tr, F) = Corr(Tr, Ts, F) * PsO2 (Ts) berechnet wird.

**Claims**

1. Device for transcutaneous determination of blood gases, comprising a transcutaneous sensor for measuring the variable of partial pressure of skin carbon dioxide (PsCO2), comprising at least one sensor for measuring the local, with respect to the transcutaneous sensor, tissue blood flow (F), and comprising a device for calculating the variable of transcutaneous partial pressure of carbon dioxide (tcpCO2) from the measured partial pressure of skin carbon dioxide (PsCO2), wherein the calculation of the variable of transcutaneous partial pressure of carbon dioxide (tcpCO2) takes into account a factor dependent on the local tissue blood flow (F), **characterized in that** the determination of the transcutaneous partial pressure of carbon dioxide (tcpCO2) is carried out taking into account the local tissue perfusion (F) and additionally the local temperature (Ts) according to the equation

$$tcpCO2(Tr, F) = \frac{PsCO2(Ts)}{10^{(Ts-Tr) \times A}} - Ms\,(Ts, F)$$

.

2. Device according to Claim 1, wherein the transcutaneous sensor for measuring the variable skin carbon dioxide partial pressure (PsCO2) is designed for measuring the variable skin oxygen partial pressure (PsO2) and wherein the device for calculating the variable transcutaneous carbon dioxide partial pressure (tcpCO2) from the measured skin carbon dioxide partial pressure (PsCO2) is designed for calculating the variable transcutaneous oxygen partial pressure (tcpO2) from the measured skin oxygen partial pressure (PsO2), wherein a factor dependent on the local tissue perfusion (f) is taken into account when calculating the magnitude of the transcutaneous oxygen partial pressure (tcpO2),
**characterized in that** the transcutaneous partial pressure of oxygen (tcpO2) is determined by taking into account the local tissue blood flow (F) according to the equation

$$tcpO2(Tr, F) = Corr(\,F) * PsO2(Ts)$$

.

3. Device according to claim 2, **characterized in that** the transcutaneous partial pressure of oxygen (tcpO2) is determined by taking into account the local temperature (Ts) according to the equation

$$tcpO2(Tr, F) = Corr(Tr, Ts, F) * PsO2(Ts)$$

.

4. Device according to any of the preceding claims, **characterized in that** at least one sensor for measuring the local tissue blood flow (F) is arranged in the same housing as for the transcutaneous sensor, and/or that sensors for measuring the local tissue blood flow (F) are configured such that they measure the local tissue blood flow (F) below the contact surface of the transcutaneous sensor.

5. Device according to any of the preceding claims, **characterized in that** sensors for measuring the local tissue blood flow (F) are part of a (photo)plethysmographic measurement system, more particularly a pulse-spectroscopic or pulseoximetric measurement system.

6. Device according to any of Claims 1 to 4, **characterized in that** sensors for measuring the local tissue blood flow (F) are part of a heating device which keeps the temperature of the contact surface constant, wherein the power supplied to the heating device is a measure of the tissue blood flow (F).

7. Device according to any of Claims 1 to 4, **characterized in that** sensors for measuring the local tissue blood flow (F) are acustic sensors or light sensors, more particularly laser sensors, and part of a Doppler measurement system.

8. Method for transcutaneous blood gas monitoring,

   wherein the variables of partial pressure of skin carbon dioxide (PsCO2) is recorded, and wherein local tissue blood flow (F) is recorded, and
   wherein a calculation device is used to calculate the variables of transcutaneous partial pressure of carbon dioxide (tcpCO2) from the measured partial pressure of skin carbon dioxide (PsCO2),
   wherein the calculation of the variables of transcutaneous partial pressure of carbon dioxide (tcpCO2) takes into account the local tissue blood flow (F) and additionally the local temperature (Ts) **characterized in that** the transcutaneous partial pressure of carbon dioxide (tcpCO2) is calculated as a function of tissue perfusion (F) according to the equation

$$tcpCO2(Tr, F) = \frac{PsCO2(Ts)}{10^{(Ts-Tr) \times A}} - Ms\,(Ts, F)$$

9. Method according to Claim 8,

   wherein the variable skin oxygen partial pressure (PsO2) is measured and wherein the variable transcutaneous oxygen partial pressure (tcpO2) is calculated from the measured skin oxygen partial pressure (PsO2) by means of the calculation device for the calculation of the variable transcutaneous carbon dioxide partial pressure (tcpCO2), wherein the variable transcutaneous oxygen partial pressure (tcpO2) is calculated taking into account the local tissue perfusion (F),
   **characterized in that** the transcutaneous partial pressure of oxygen (tcpO2) is calculated as a function of the tissue blood flow (F) according to the equation

$$tcpO2(Tr, F) = Corr(Tr, Ts, F) * PsO2(Ts)$$

10. Method according to Claim 9, **characterized in that** the transcutaneous partial pressure of oxygen (tcpO2) is additionally calculated as a function of the local temperature (Ts) according to the equation

$$tcpO2(Tr, F) = Corr(Tr, Ts, F) * PsO2(Ts)$$

**Revendications**

1. Dispositif pour la détermination transcutanée de gaz sanguins comprenant un capteur transcutané pour la mesure de la grandeur pression partielle de dioxyde de carbone dans la peau (PsCO2), comprenant au moins un capteur pour la mesure de l'irrigation sanguine locale dans le tissu (F) par rapport au capteur transcutané, et comprenant un dispositif pour le calcul de la grandeur pression partielle transcutanée de dioxyde de carbone (tcpCO2) à partir de la pression partielle de dioxyde de carbone mesurée dans la peau (PsCO2), un facteur dépendant de l'irrigation sanguine locale dans le tissu (F) étant pris en considération lors du calcul de la grandeur pression partielle transcutanée de dioxyde de carbone (tcpCO2) **caractérisé en ce que** la détermination de la pression partielle de dioxyde de carbone transcutanée (tcpCO2) s'effectue en tenant compte de l'irrigation sanguine locale des tissus (F) et en plus de la température locale (Ts) conformément à l'équation

$$tcpCO2(Tr, F) = \frac{PsCO2(Ts)}{10^{(Ts-Tr) \times A}} - Ms\,(Ts, F)$$

2. Dispositif selon la revendication 1, dans lequel le capteur transcutané pour la mesure de la grandeur de la pression partielle de dioxyde de carbone de la peau (PsCO2) étant conçu pour mesurer la grandeur de la pression partielle d'oxygène de la peau (PsO2) et le dispositif pour le calcul de la grandeur de la pression partielle de dioxyde de carbone transcutanée (tcpCO2) à partir de la pression partielle de dioxyde de carbone de la peau (PsCO2) mesurée étant conçu pour calculer la grandeur de la pression partielle d'oxygène transcutanée (tcpO2) à partir de la pression partielle d'oxygène de la peau (PsO2) mesurée, un facteur dépendant de l'irrigation sanguine locale des tissus (f) étant pris en compte lors du calcul de la grandeur de la pression partielle transcutanée d'oxygène (tcpO2), **caractérisé en ce que** la détermination de la pression partielle transcutanée d'oxygène (tcpO2) est réalisée en prenant en considération l'irrigation sanguine locale dans le tissu (F) selon l'équation

$$tcpO2(Tr, F) = Corr(F) * PsO2(Ts)$$

3. Dispositif selon la revendication 2 , **caractérisé en ce que** la détermination de la pression partielle transcutanée d'oxygène (tcpO2) est réalisée en prenant en considération la température locale (Ts) selon l'équation

$$tcpO2(Tr, F) = Corr(Tr, Ts, F) * PsO2(Ts)$$

4. Dispositif selon l'une quelconque des revendications précédentes, **caractérisé en ce qu'**au moins un capteur pour la mesure de l'irrigation sanguine locale dans le tissu (F) est disposé dans le même boîtier que le capteur transcutané et/ou **en ce que** les capteurs pour la mesure de l'irrigation sanguine locale dans le tissu (F) sont conçus de manière telle qu'ils mesurent l'irrigation sanguine locale dans le tissu (F) sous la surface d'appui du capteur transcutané.

5. Dispositif selon l'une quelconque des revendications précédentes, **caractérisé en ce que** les capteurs pour la mesure de l'irrigation sanguine locale dans le tissu (F) font partie d'un système de mesure (photo)pléthysmographique, en particulier un système de mesure par spectroscopie pulsée ou oxymétrie pulsée.

6. Dispositif selon l'une quelconque des revendications 1 à 4, **caractérisé en ce que** les capteurs pour la mesure de l'irrigation sanguine locale dans le tissu (F) font partie d'un dispositif de chauffage, qui maintient constante la température de la surface d'appui, la puissance alimentée au dispositif de chauffage étant une mesure de l'irrigation sanguine dans le tissu (F).

7. Dispositif selon l'une quelconque des revendications 1 à 4, **caractérisé en ce que** les capteurs pour la mesure de l'irrigation sanguine locale dans le tissu (F) sont conçus sous forme de capteurs acoustiques ou de capteurs de lumière, en particulier de capteurs au laser, et forment une partie d'un système de mesure Doppler.

8. Procédé pour la surveillance transcutanée des gaz sanguins, la grandeur pression partielle de dioxyde de carbone dans la peau (PsCO2) étant enregistrée, et une irrigation sanguine locale dans le tissu (F) étant enregistrée, la grandeur pression partielle transcutanée de dioxyde de carbone (tcpCO2) étant calculée au moyen d'un dispositif de calcul à partir de la pression partielle de dioxyde de carbone mesurée dans la peau (PsCO2), en tenant compte, lors du calcul de la grandeur pression partielle transcutanée de dioxyde de carbone (tcpCO2), de l'irrigation sanguine locale dans le tissu (F) et en plus de la température locale (Ts), **caractérisé en ce que** la pression partielle de dioxyde de carbone transcutanée (tcpCO2) est calculée en fonction de la perfusion tissulaire (F) selon l'équation

$$tcpCO2(Tr, F) = \frac{PsCO2(Ts)}{10^{(Ts-Tr) \times A}} - Ms(Ts, F)$$

.

9. Procédé selon la revendication 8, dans lequel la grandeur de la pression partielle d'oxygène de la peau (PsO2) est saisie et dans lequel, avec le dispositif de calcul pour le calcul de la grandeur de la pression partielle transcutanée de dioxyde de carbone (tcpCO2) à partir de la pression partielle mesurée de dioxyde de carbone de la peau (PsCO2), la grandeur de la pression partielle transcutanée d'oxygène (tcpO2) est calculée à partir de la pression partielle mesurée d'oxygène de la peau (PsO2), la circulation sanguine tissulaire locale (F) étant prise en compte lors du calcul de la valeur de la pression partielle transcutanée d'oxygène (tcpO2), **caractérisé en ce que** la pression partielle transcutanée d'oxygène (tcpO2) est calculée en fonction de l'irrigation sanguine dans le tissu (F) selon l'équation

$$tcpO2(Tr, F) = Corr(Tr, Ts, F) * PsO2(Ts)$$

10. Procédé selon la revendication 9, **caractérisé en ce que** la pression partielle transcutanée d'oxygène (tcpO2) est en outre calculée en fonction de la température locale (Ts) selon l'équation

$$tcpO2(Tr, F) = Corr(Tr, Ts, F) * PsO2(Ts)$$

Fig.1

Fig.2

Figur 3

Figur 4

Figur 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0241770 A
- GB 1461345 A
- US 20050277818 A1